# EUROPEAN PATENT APPLICATION

(11) **EP 0 910 992 A2**
(43) Date of publication of application: **28.04.1999**
(21) Application number: 98830604.9
(22) Date of filing: 13.10.1998
(51) Int. Cl.: A61B 17/39, A61C 3/00

(54) **A radio scalpel.**

(30) Priority: 15.10.1997 IT BO970618
(71) Applicant: CASTELLINI S.p.A., I-40013 Castelmaggiore (Bologna) (IT)
(72) Inventor: Castellini, Franco, 40124 Bologna (IT)
(74) Representative: Lanzoni, Luciano

(57) **Abstract**

A radio scalpel envisages a grip body (2) which houses lighting means (7) for an attachment (4), the means extending along a second axis (X''), powered by a utility circuit (10), their beam of light arriving at a front end (2a) of the body (2) through means (8) for the passage of the beam of light; the latter are operated upon by means (9) for adjusting the direction (D) of the beam of light, relative to the second axis (X''), depending on the type of attachment (4) fitted, so as to obtain a beam of light which is always directed at an operating end (4a) of the attachment.

## Description

The present invention relates to a radio scalpel, in particular for application in the dental sector for surgical operations.

Within the range of handpieces used in the dental sector (reference is made to this particular application), the so-called electronic radio scalpel is one of those handpieces used only in special, particularly delicate surgical operations carried out in the mouth and, in particular, on gengival tissue.

As is known, the radio scalpel consists of a cylindrical body, which forms a grip to be held by the dental surgeon and houses known means for the passage of a high frequency current (around 1 MHz), which is guided from a neutral electrode (held by the patient) to an operating electrode defined by the scalpel that can be fitted to one end of the body of the handpiece and connected to the above-mentioned means for passage of the current. A sheath protrudes from the other end of the handpiece body, protecting the wires which connect the above-mentioned means for passage of the current to devices which generate the high frequency current and control systems (for example, a microprocessor) located on the dental chair.

The microprocessor can be used to control generation of the high frequency current and its modulation, to obtain a series of operating functions: a coagulating effect in the zone in question, through a moderate increase in the temperature of the tissue cell structure; a cutting effect, through a sharp increase in temperature and evaporation of the cell structure, or a cut with coagulation, through a modulation of the current such that the variation in the temperature of the cell tissue is controlled.

Such a radio scalpel, in contrast to other, conventional handpieces used regularly during normal dental work (such as turbo-drills, syringes, tartar removers, etc.), is not fitted with a light of its own, that is to say, directly on the handpiece body. The above-mentioned conventional handpieces have been fitted with their own lights for some years now, for practical purposes, since normal lights outside the mouth illuminate a much wider zone than that in which the dental surgeon needs to operate and do not always suitably reach, for example, deep cavities.

Moreover, in the case of conventional lights with bulbs, the light source may be blocked (even partially) by the dental surgeon whilst operating in the patient's mouth, thus reducing visibility in the field of operation.

The widespread use of handpieces fitted with their own lights also discourages the dental surgeon's familiarity with the use of other light sources, so that when the dental surgeon must carry out an operation which is delicate in itself, he/she cannot always rely on optimum operating conditions.

Although these disadvantages have been known for some time, until now the radio scalpel has never been fitted with any kind of light: the use of conventional light sources (that is to say, with optic fibres, as in the conventional handpieces) presents a significant problem, since the operating end of the scalpel (technically known as the "attachment") which may be used and fitted to the handpiece body comes in different shapes and sizes (depending on the operation to be carried out), meaning that the zone to be lit at the end of the handpiece is very large and difficult to light with the above-mentioned optic fibre systems: in fact, the latter have a reduced lighting zone, directed towards a preset point and substantially coinciding with the operating tip of the tool used (almost always of the same size and shape).

For this reason, the Applicant has designed and made a radio scalpel equipped with its own lighting unit, structured in such a way that it can be adapted to the various configurations of the scalpels fitted on the handpiece body, and with rapid, safe adjustment of the light source, which does not compromise the reliability of the handpiece.

The technical features of the invention, according to the above-mentioned objects, are clearly described in the claims herein and their advantages are made more evident in the detailed description which follows, with reference to the accompanying drawings which illustrate a preferred embodiment of the invention, without limiting the scope of its application, and in which:
- Figure 1 is a side view of a radio scalpel according to the present invention, with some parts shown in cross-section to better illustrate others;
- Figure 2 is a scaled-up detail of the radio scalpel illustrated in Figure 1, with some parts shown in cross-section to better illustrate others;
- Figure 3 illustrates a section III-III illustrated in Figure 2;
- Figure 4 is a scaled-up side view of an alternative embodiment of a detail of the radio scalpel, with some parts cut away to better illustrate others.

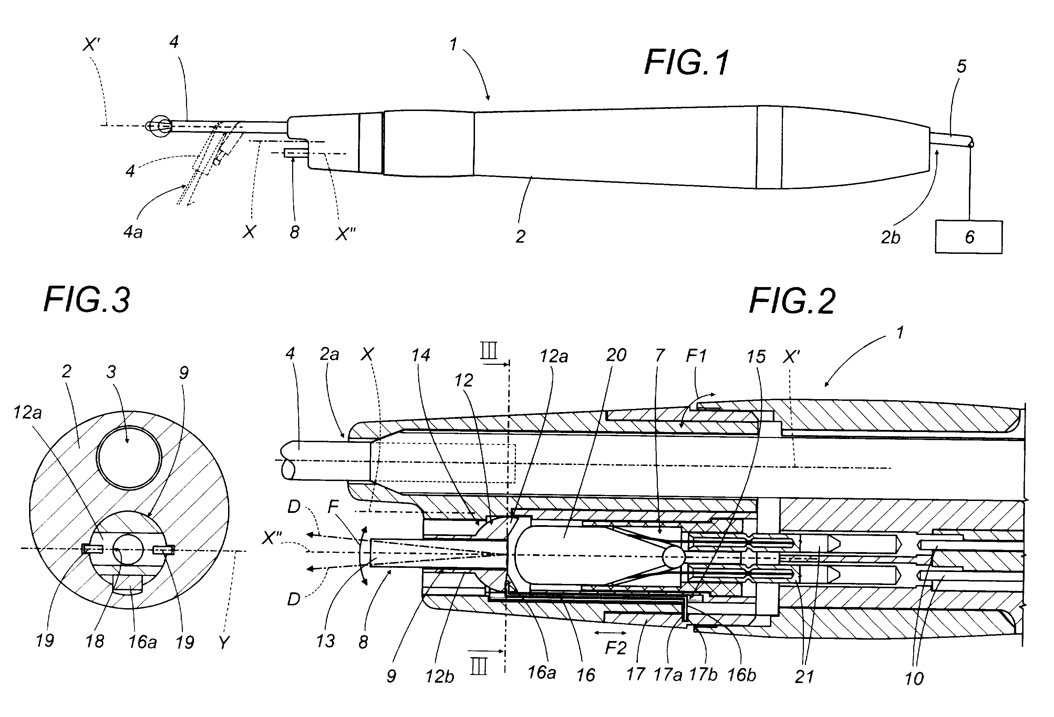

With reference to the accompanying drawings, and in particular Figure 1, the dental radio scalpel concerned, which operates with a high frequency current, is used to perform surgery in the mouth. This radio scalpel, indicated as a whole by the numeral 1, comprises a body 2 which forms the grip of the handpiece. The body 2 is cylindrical and, at a front end 2a, is fitted with means 3 for the attachment of one or more tools or attachments 4 of different shapes and sizes (some of which are straight rods, others partially angled downwards, and almost all project from the handpiece and have different operating ends).

These attachments 4 are illustrated in Figures 1, 2 and 4, overlapping one another in order to make the structure of the handpiece clearer in the description which follows.

The above-mentioned attachment means 3 extend along a first axis X' parallel with the longitudinal axis X of the body 2 and are electrically connected to control means 6 for the attachment 4 fitted to the body 2. The control means are of the known type and, therefore, schematically illustrated as a block outside the handpiece 1.

A sheath 5 projects from the rear end 2b of the body 2, protecting the wires for connection to the above-mentioned control means 6.

Means 7 for lighting the attachment 4, that is to say, its operating end 4a, are envisaged in the body.

The lighting means 7 comprise a bulb 20, positioned horizontally inside the grip body 2 and extending, together with connectors 21 for the power supply and connection to a utility circuit 10 of the known type, along a second axis X'' parallel with the longitudinal axis X. In the accompanying drawings, the bulb 20 is located near the front end 2a of the grip body 2, although if construction requirements necessitate it, the bulb may be fitted near the rear end of the body 2 and have optic fibres which allow the beam of light to pass to the front end 2a.

The beam of light generated by this bulb 20, therefore, arrives at the front end 2a of the body 2 through means 8 for the passage of the beam of light.

These means 8 for the passage of the beam of light may be adjusted using means 9 which adjust the direction of the beam (labelled D), relative to the above-mentioned second longitudinal axis X'', depending on the type of attachment 4 fitted: in this way, the beam of light is always directed at the operating end 4a of the attachment.

More specifically, as shown in Figures 1 to 3, the means 9 for adjusting the direction D of the beam of light may comprise a mobile support 12 for a known optic fibre 13, the latter defining the above-mentioned means 8 for passage of the beam of light.

The support 12 is housed in the body 2 in a seat 14, made at the front end 2a, so as to allow part of the optic fibre 13 to exit said end. A first portion 12a of the support 12 is attached to the body 2 in such a way that it can rotate about an axis Y (see arrow F in Figures 2 and 3) perpendicular to the second longitudinal axis X'', thus allowing the optic fibre 13 to be angled in either direction relative to the second axis X'', depending on the type of scalpel 4 used.

More precisely, the support 12 consists of the half-sphere shaped first portion 12a, which is partially attached to a second, cylindrical portion 12b, which defines the actual support for the optic fibre 13 housed in a central through-seat 18 made coaxially on the two portions 12a and 12b.

To obtain the above-mentioned rotation, the first portion 12a is attached to a pair of pins 19 (see Figure 3), radially opposite one another on the first portion, which coincide with the transverse axis Y and are, in turn, attached to the body 2 in such a way as to define two points for the guided rocking of the support 12.

Cam means 15 act upon the first portion 12a. The cam means may be activated from outside the body 2 to adjust the angle of the optic fibre 13.

The cam means 15 comprise a flat rod 16 inserted in the grip body 2, extending parallel with the second longitudinal axis X'', its ends connected to the first portion 12a of the support 12, by an angled projection 16a, and respectively a ring nut 17 which can be screwed onto the outer circumference of the body 2, by means of a second angled projection 16b.

The surfaces 17a and 17b of the ring nut 17 which make contact with the second projection 16b may be shaped so that, when the ring nut rotates in either direction (see arrow F1 in Figure 2), the rod 16 can slide (see arrow F2) in the two directions which correspond to the pushing or pulling of the flat rod corresponding to a different angle of the support 12, thus obtaining a different angle of the direction D of the beam of light.

The outer surface of the ring nut 17 may bear a set of reference symbols (not illustrated), relative to a fixed notch on the body 2, which display the angle of the beam of light at that moment: in this way the dental surgeon is always aware of the position of the support 12, without having to test the handpiece.

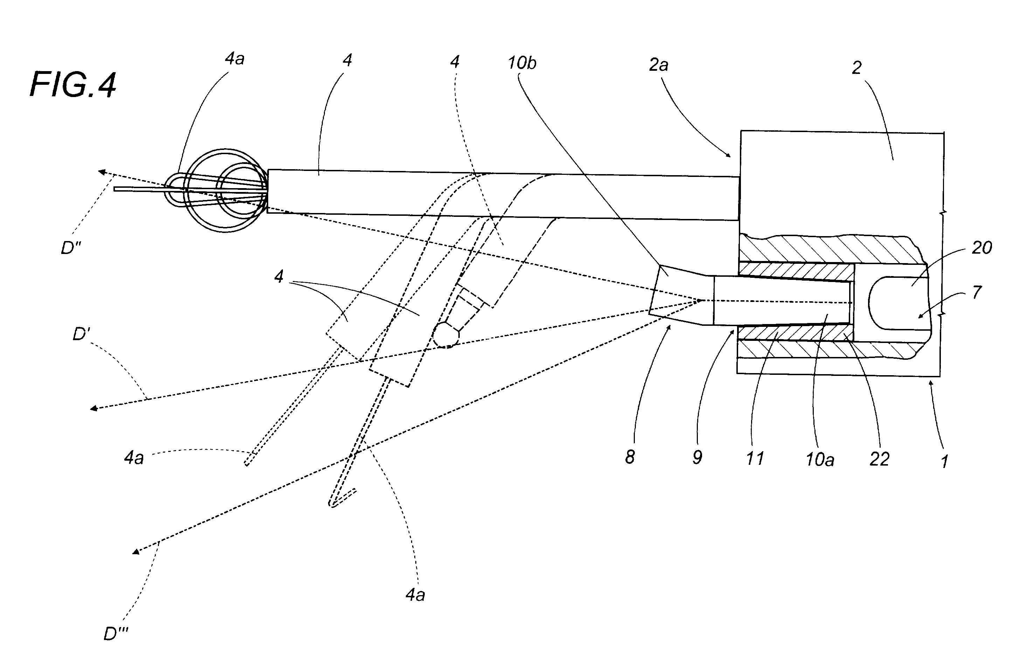

Figure 4 illustrates a simplified alternative embodiment of the means 9 for adjustment of the direction D of the beam of light. In this embodiment an interchangeable optic fibre tube is used, also defining the means 8 for passage of the beam of light, and substantially extending in two parts: one part, labelled 10a, is coaxial with the second axis X'' and can be attached from the outside at the front end 2a of the body 2; the other part of the tube, labelled 10b, is located outside the body 2 and is at an angle to the second axis X'', so as to direct the beam of light in one or more directions, labelled D', D'', D''', depending on the type of attachment 4 used.

More precisely, the first portion 10a can press fitted in a seat 11, that is, fitted by interference, for example, in a bushing 22 with truncated-cone shaped cross-section, and in such a way that it is opposite the bulb 20 inserted in the body 2.

Obviously, there may be various types of optic fibres, ready-fitted for use with the corresponding scalpels that can be used: for example, if the scalpels are numbered and colour-coded, the optic fibres may have similar marks or references, so that the dental surgeon can rapidly attach the correct optic fibre for the selected attachment without the need for special tests.

The scalpel structure described, therefore, achieves the preset objects with an extremely simple, independent lighting solution which does not weigh down the handpiece. It is very versatile according to the attachment used, thanks to the possibility of precision adjustments which do not alter the original operating and reliability characteristics of the instrument, instead improving its operating quality.

The present invention may be subject to numerous modifications and variations, all encompassed by the original design concept. Moreover, all components may be replaced with technically equivalent parts.

## Claims

1. A radio scalpel for surgical operations, the radio scalpel (1) being of the type comprising a cylindrical grip body (2) having attachment means (3) at the front end (2a), said attachment means extending along a first axis (X') parallel with the longitudinal axis (X) of the body (2) and allowing the attachment of a plurality of tools or attachments (4) with operating ends (4a) of varying shapes and sizes, the radio scalpel being characterised in that the grip body (2) houses lighting means (7) for the attachment (4), said means extending along a second axis (X''), being powered by a utility circuit (10) and whose beam of light arrives at the front end (2a) of the body (2), means (9) for adjusting the direction (D) of the beam of light being envisaged, acting upon the lighting means (7) in such a way that the position of the second longitudinal axis (X'') is varied according to the type and shape of the attachment (4) fitted, so as to obtain a beam of light that is always directed at the operating end (4a) of the attachment.

2. The radio scalpel according to claim 1, characterised in that means (8) are envisaged for the passage of the beam of light generated by the lighting means (7) towards the operating end (4a) of the attachment (4).

3. The radio scalpel according to claim 1, characterised in that the second, longitudinal axis (X'') of the lighting means (7) is parallel with the longitudinal axis (X) of the grip body (2).

4. The radio scalpel according to claim 1, characterised in that the means (9) for adjusting the direction (D) of the beam of light comprise an interchangeable optic fibre tube, also defining the means (8) for the passage of the light beam, and extending substantially in two parts, one part (10a) being coaxial with the second axis (X''), it being possible to attach this part from the outside at the front end (2a) of the body (2), and the other part (10b) being positioned at an angle to the second axis (X'') and in a direction (D', D'', D''') depending on the type of attachment (4) used; it being possible to stably house said first portion (10a), by a press fit, in a seat (11) on the body (2) inside which it arrives at the lighting means (7).

5. The radio scalpel according to claim 1, characterised in that the means (9) for adjusting the direction (D) of the beam of light comprise a support (12) for an optic fibre (13), defining the means (8) for the passage of the beam of light, being housed in the body (2) in a seat (14) made at the front end (2a) so as to allow the optic fibre (13) to exit said end; the support (12) having a first portion (12a) attached to the body (2) in such a way that it rotates about an axis (Y) perpendicular to the second longitudinal axis (X''), allowing the optic fibre (13) to be angled in either direction relative to the second axis (X''), depending on the type of attachment (4) used; there being cam means (15), operating on the first portion (12a), it being possible to activate the cam means from outside the body (2), so that the optic fire (13) can be angled.

6. The radio scalpel according to claim 5, characterised in that the cam means (15) comprise a flat rod (16) inserted in the grip body (2), the rod extending parallel with the second longitudinal axis (X''), its ends being attached respectively to the first portion (12a) of the support (12) and to a ring nut (17) which may be screwed onto the outer circumference of the body (2), being designed so that when it rotates, in either direction, it allows the rod (16) to slide, again in either direction, corresponding to a pushing or pulling action on the flat rod (16) sufficient to obtain said angling of the support (12).

7. The radio scalpel according to claim 5, characterised in that the support (12) comprises the first portion (12a), in the shape of a half-sphere and partially joined to a cylindrical second portion (12b) defining the support for the optic fibre (13) housed in a central through-seat (18) made coaxially on the two portions (12a, 12b); the first portion (12a) being attached to a pair of pins (19) located radially opposite one another on the first portion and designed to allow its rotation.
